# FASCICULE DE BREVET EUROPEEN

(11) **EP 0 538 353 B1**
(45) Date de publication et mention de la délivrance du brevet: **12.06.1996**
(21) Numéro de dépôt: 91913033.6
(22) Date de dépôt: 11.07.1991
(51) Int. Cl.: C12N 15/31, C07K 14/255, C12Q 1/68, G01N 33/569

(54) **SEQUENCES NUCLEIQUES ISSUES DU GENOME DE $i(SALMONELLA) TYPHI, ET LEURS UTILISATIONS NOTAMMENT POUR LE DIAGNOSTIC $i(IN VITRO) DE LA PRESENCE DE BACTERIES DU GENRE $i(SALMONELLA) DANS LES PRODUITS ALIMENTAIRES**
SALMONELLA GENOM NÜKLEINSÄURE, IHRE VERWENDUNG, BESONDERS BEI DER IN VITRO DIAGNOSE DER ANWESENHEIT VON BAKTERIEN DER GATTUNG SALMONELLA IN LEBENSMITTELN
NUCLEIC SEQUENCES FROM THE GENOME OF SALMONELLA TYPHI, AND USES THEREOF PARTICULARLY FOR THE IN VITRO DIAGNOSIS OF THE PRESENCE OF SALMONELLA BACTERIA IN FOODSTUFFS

(30) Priorité: 11.07.1990 FR 9008852
(43) Date de publication de la demande: 28.04.1993
(60) Demande divisionnaire: 95116846.7
(73) Titulaire: INSTITUT PASTEUR, F-75724 Paris Cédex 15 (FR); INSTITUT NATIONAL DE LA SANTE ET DE LA RECHERCHE MEDICALE (INSERM), 75654 Paris Cédex 13 (FR)
(72) Inventeur: POPOFF, Michel, Yvan, F-78370 Plaisir (FR); DION, Michel, Pierre, F-75013 Paris (FR)
(74) Mandataire: Desaix, Anne
(86) Numéro de dépôt international: FR9100564
(87) Numéro de publication internationale: WO9201056

(56) Documents cités:
- EP-A- 0 114 668
- MICROBIAL PATHOGENESIS, vol. 6, Academic Press, Harcourt Jovanovich Publishers, février 1989; M.J. MROCZENSKI-WILDEY, pp. 143-152
- PROCEEDINGS OF THE NATL. ACADEMY OF SCIENCES USA, vol. 86, août 1989, Washington, DC (US); J.E. GALAN et al., pp. 6383-6387
- MICROBIOLOGY & IMMUNITY, vol. 31, no. 1, 1987; H. YOKOHAMA et al., pp. 1- 11

## Description

La présente invention a pour objet des séquences nucléiques issues du génome de Salmonella Typhi, et leurs utilisations notamment pour le diagnostic in vitro de la présence de bactéries du genre Salmonella dans un échantillon biologique susceptible de les contenir, et plus particulièrement dans les produits alimentaires.

Les produits alimentaires prennent actuellement une importance grandissante. Pour des motifs sociaux, les denrées transformées, prêtes à la consommation, ne cessent de susciter une demande accrue de la part des acheteurs. Il en résulte que les sources de production et la présentation de ces produits se sont considérablement modifiées durant les quinze dernières années (fabrication de masse dans des usines spécialisées ; commercialisation en unités conditionnées, généralement sous pellicule plastique).

Pour des raisons de santé publique, la technologie des fabrications et les produits eux-mêmes sont soumis à une surveillance hygiénique de plus en plus stricte. Dans le cadre des contrôles bactériologiques, les bactéries responsables de toxi-infections alimentaires sont recherchées systématiquement, et parmi celles-ci, ce sont les Salmonella qui sont concernées en priorité. Les normes de salubrité sont d'ailleurs très claires pour ce genre bactérien : absence de Salmonella dans 25 grammes de produit.

Du point de vue de la taxonomie, le genre Salmonella appartient à la famille des Enterobacteriaceae. Ce genre comprend une seule et unique espèce, S.enterica, qui peut être subdivisée en sept sous-espèces. A l'intérieur de chacune de ces sous-espèces, on peut individualiser un grand nombre de sérotypes à l'aide de sérums dirigés contre les antigènes O polysaccharidiques et les antigènes H flagellaires. En 1989, on connaissait 2267 sérotypes de Salmonella.

Les Salmonella sont des bactéries entéro-in-vasives qui sont pathogènes pour l'homme et les animaux. Leur pouvoir pathogène est lié à leur capacité d'envahir l'épithélium intestinal. Cette étape peut se limiter à la muqueuse dans le cas d'une toxi-infection par exemple, ou être suivie d'une dissémination systémique (cas de la fièvre thyphoïde). La contamination de l'hôte par Salmonella a lieu par voie buccale dans la très grande majorité des cas. Ceci explique pourquoi les Salmonella sont recherchées systématiquement dans le cadre de contrôles bactériologiques d'échantillons biologiques.

La méthode de référence de recherche systématique de Salmonella, recommandée par l'Association Française de Normalisation (AFNOR), comprend : la mise en culture du produit à analyser dans deux milieux d'enrichissement différents, incubés à deux températures différentes et isolés sur deux milieux sélectifs différents (cette étape est souvent précédée d'une étape de "revivification" dans un bouillon nutritif) ; repiquage de 6 colonies au minimum sur milieux d'identification rapide ; enfin typage sérologique de la souche. Si le protocole AFNOR doit être scrupuleusement suivi lors d'une expertise officielle, on conçoit qu'il soit difficilement applicable lors de contrôles systématiques en raison du temps nécessaire pour effectuer l'examen (au minimum une semaine) et de son prix de revient.

Des nouvelles méthodes de recherche de Salmonella reposent soit sur des tests enzymatiques, soit sur l'utilisation de sondes nucléiques. Il faut souligner que dans les deux cas, une étape de culture en milieu riche et une subculture dans un milieu d'enrichissement sont recommandées, voire indispensables.

Les tests immuno-enzymatiques qui utilisent des anticorps monoclonaux, sont aisés à mettre en oeuvre, donnent des résultats en quatre à six heures et sont d'un prix de revient abordable. Leur gros inconvénient réside dans le fait qu'ils donnent souvent de fausses réactions positives et parfois de fausses réactions négatives. Les conséquences de ces fausses réactions sont importantes dans les deux cas : s'il s'agit d'une fausse réaction positive, il y aura saisie du produit et mise en route par le laboratoire d'analyse d'un processus pour isoler une Salmonella qui n'existe pas; s'il s'agit d'une fausse réaction négative, le produit sera commercialisé avec les risques que cela comporte pour la santé publique.

Une étude récente sur 250 souches de Salmonella et 75 souches bactériennes n'appartenant pas au genre Salmonella, a abouti à 17 fausses réactions positives et 2 fausses réactions négatives (D'AOUST, J.Y. (1987): "Efficacité de la trousse immunoenzymatique BIOENZABEAD pour le dépistage de Sallnonella spp. dans les aliments", Microorganismes et Aliments : Technique Rapide, Contrôle industriel. Colloque de la Société Française de Microbiologie, Paris). Cet essai ayant été effectué sur des cultures pures, on peut penser que le nombre de fausses réactions aurait été plus important avec des produits poly-microbiens (compétition microbienne ; risque de réactions antigéniques croisées).

Les sondes nucléiques sont constituées par un fragment d'ADN génomique (FITTS, R. et al (1983), "DNA-DNA Hybridization Assay For Detection Of Salmonella spp". In Foods, Applied and Environmental Microbiology, 46, 1146-1151). Elles sont réputées spécifiques. Il existe plusieurs trousses (ou kits) commercialisées. En fait, il apparaît que ces sondes présentent deux inconvénients majeurs : leur manque de spécificité selon les résultats communiqués par des utilisateurs (réaction croisée avec les Citrobacter, par exemple) et leur manque de sensibilité- (seuil limite de détection : 10⁵ Salmonella ; ou selon certains auteurs, 2,5 µg d'ADN soit environ 10⁷ bactéries).

La présente invention a précisément pour objet des séquences nucléiques utilisables en tant que sondes nucléiques pour la détection de Salmonella, ces sondes étant parfaitement spécifiques du genre Salmonella (pas de réaction croisée avec d'autres bactéries, notamment avec les bactéries du genre Citrobacter).

L'invention a également pour objet l'utilisation de ces sondes nucléiques dans des méthodes de détection ou de dosage in vitro de Salmonella présentant les avantages suivants :
- forte sensibilité,
- réponse rapide ; le résultat de l'analyse peut être rendu dans les 48 heures, voire 24 heures,
- mise en oeuvre aisée, notamment sans utilisation d'un produit radioactif,
- prix de revient raisonnable.

Salmonella sous-espèce enterica sérotype Typhi (bactérie désignée par Typhi ci-après) est l'agent de la fièvre typhoïde humaine. Cette bactérie est strictement pathogène pour l'homme. A la suite d'une contamination par voie buccale, Typhi va franchir la barrière intestinale pour atteindre les ganglions mésentériques grâce à un système génétique lui permettant d'adhérer et de pénétrer dans les cellules épithéliales de la muqueuse intestinale. Faute de modèle expérimental, cette première étape de l'infection est étudiée in vitro sur cellules HeLa en culture, car Typhi est capable d'ahérer et de pénétrer dans ces cellules.

L'invention a précisément pour objet toute séquence nucléique caractérisée en ce qu'elle comporte toute ou partie de l'information génétique nécessaire à l'activité d'infection in vitro des cellules HeLa en culture par les bactéries du genre Salmonella.

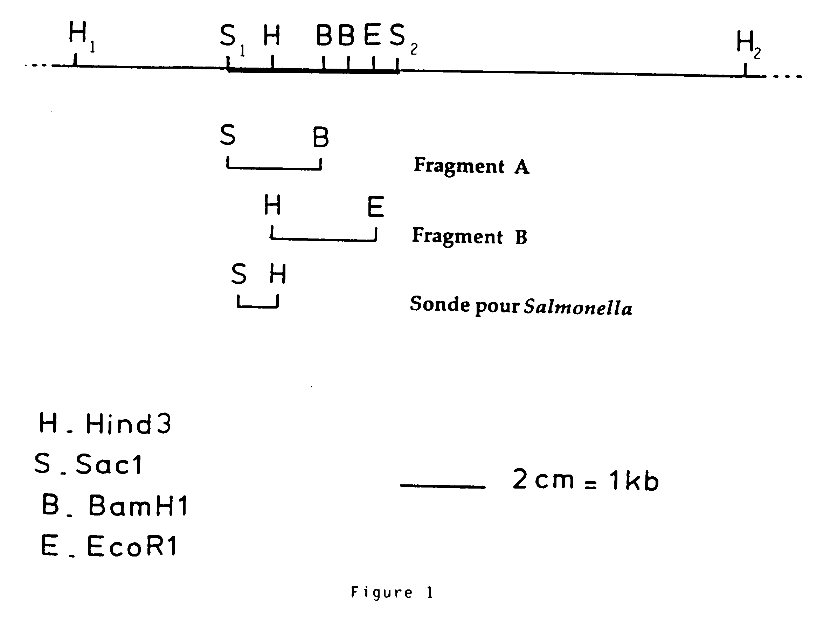

L'invention a plus précisément pour objet toute séquence nucléique telle que définie ci-dessus, et comprenant toute ou partie de la séquence de 7,9 kb délimitée par les deux sites HindIII, désignés par H₁ et H₂ sur la carte de restriction de ladite séquence représentée sur la figure 1.

Les séquences nucléiques de l'invention sont plus particulièrement celles répondant aux caractéristiques définies ci-dessus, et qui sont issus du génome de la souche Ty 2 de Typhi déposée à la Collection de l'Institut Pasteur sous le n° CIP 55-35.

L'invention concerne plus particulièrement toute séquence nucléique issue du génome de la souche Ty 2 sus-mentionnée, cette séquence comportant toute ou partie de l'information génétique définie ci-dessus, et comprenant toute ou partie de la séquence de 2 kb délimitée par les deux sites SacI, désignés par S₁ et S₂ sur la carte de restriction représentée sur la figure 1.

D'une manière plus générale, l'invention concerne toute séquence nucléique comportant toute ou partie de l'information génétique nécessaire à l'activité d'infection in vitro des cellules HeLa en culture par les bactéries du genre Salmonella, et susceptible de s'hybrider avec toute ou partie de l'une au moins des séquences nucléiques définies ci-dessus, dans des conditions stringentes. Ces conditions stringentes sont les suivantes : 65°C durant 18 heures en 6 x SSC (lxSSC est constitué de 0,15M Nacl et 0,015M citrate trisodique à pH 7) en milieu de Denhardt (0,1% Ficoll ; 0,1 % de polyvynil-pyrrolidone ; 0,1 % de sérum albumine de boeuf).

L'invention concerne également toute séquence nucléique comprise dans l'une des séquences nucléiques définies ci-dessus, ou susceptible de s'hybrider avec l'une de ces séquences dans les conditions d'hybridation définies ci-dessus, et étant utilisable en tant que sonde pour la mise en oeuvre d'un procédé de détection in vitro de bactéries du genre Salmonella, et plus particulièrement des Salmonella pathogènes telles que Typhi, susceptibles d'adhérer et de pénétrer dans les cellules épithéliales de la muqueuse intestinale dans l'organisme.

Avangateusement les sondes nucléiques de l'invention sont constituées d'une succession d'environ 200 à 500 nucléotides.

A ce titre, l'invention a plus particulièrement pour objet une sonde nucléique caractérisée par l'enchaînement nucléotidique (I) suivant :

1
5'> CAATTACGCCCCGATTATTATATCTCCGGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGGACACAAAGCAAATTAGCGAGCTA
AATTCGATTGACAGTACCATGGTCTACTTACGCGGTAAGCATGAG
TTAAATCAATACACCCCCTATAGCTTACAGCAAGCGCTTAAATTG
CTGACTCAATGCGTTAATATGTCGCCAAACAGCATTGCGCCTTAC
TGTGCGCTGGCAGAATGCTACCTCAGCATGGCGCAAATGGGGATT
TTTGATAAACAAAACGCAATGATCAAAGCTAAAGAACATGCGATT
AAGGCGACAGAGCTGGACCACAATAATCCACAAGCTT < 3'
587

L'invention concerne également toute séquence nucléique comprise dans l'une des séquences nucléiques définies ci-dessus, ou susceptible de s'hybrider avec l'une de ces séquences dans les conditions d'hybridation définies ci-dessus, et étant utilisable en tant qu'amorce nucléique pour l'amplification génique d'une des séquences nucléiques de l'invention.

Avantageusement les amorces nucléiques de l'invention sont constituées d'une succession d'environ 10 à 30 nucléotides.

Les techniques d'amplification génique sont d'un appoint considérable pour la mise au point de méthodes de diagnostic in vitro particulièrement sensibles. Parmi ces techniques d'amplification génique, on peut citer la technique PCR (Polymerase Chain Reaction) telle que décrite dans les demandes de brevet européen n° 86/302.298.4 du 27/03/1986 et n° 87/300.203.4 du 09/01/1987, ou encore la technique dite "Qβreplicase" décrite dans Biotechnology, vol.6, page 1197 (octobre 1988) et celle procédant à l'aide d'une ARN polymérase (T7RNA polymérase) décrite dans la demande de brevet international n° WO89/01050. Ces techniques permettent d'améliorer la sensibilité de détection des acides nucléiques des virus ou des bactéries, et nécessitent l'utilisation d'amorces de synthèse spécifiques.

A ce titre, l'invention a plus particulièrement pour objet les amorces nucléiques SS-1 et SS-2 suivantes : 
SS-1
5'>GGT CCA GCT CTG TCG CC<3'
 
SS-2
5'>CCG GGC AGA TGA TAC CC<3'
 utilisables pour l'amplification du nombre de copies de la séquence nucléique délimitée par les nucléotides situés aux positions 26 et 469 de l'enchaînement nucléotidique (I) de l'invention.

Font également partie de l'invention, les acides nucléiques variants par rapport à la séquence nucléique (I) ou aux amorces SS-1 et SS-2 sus-définies et qui comportent certaines mutations localisées dans la mesure où ces acides nucléiques variants s'hybrident avec la séquence (I) ou les amorces nucléiques précédemment définies dans les conditions d'hybridation définies ci-dessus dans la description.

L'invention a également pour objet tout peptide ou polypeptide correspondant selon le code génétique universel à une séquence nucléique de l'invention.

A ce titre l'invention concerne plus particulièrement tout polypeptide constitué par toute ou partie de la séquence en acides aminés (II) suivante, cette séquence correspondant selon le code génétique universel à la séquence nucléique (I) décrite ci-dessus :

GlnLeuArgProAspTyrTyrIleSerGlyGlnMetIleProAsp
GlyAsnAspAsnIleValGlnIleGluIleValArgValLysGly
TyrHisLeuLeuHisGlnGluSerIleLysLeuIleGluHisGln
ProAlaSerLeuLeuGlnAsnLysIleAlaAsnLeuLeuLeuArg
CysIleProGlyLeuArgTrpAspThrLysGlnIleSerGluLeu
AsnSerIleAspSerThrMetValTyrLeuArgGlyLysHisGlu
LeuAsnGlnTyrThrProTyrSerLeuGlnGlnAlaLeuLysLeu
LeuThrGlnCysValAsnMetSerProAsnSerIleAlaProTyr
CysAlaLeuAlaGluCysTyrLeuSerMetAlaGlnMetGlyIle
PheAspLysGlnAsnAlaMetIleLysAlaLysGluHisAlaIle
LysAlaThrGluLeuAspHisAsnAsnProGlnAla

Il va de soi que toute séquence peptidique issue de la modification, par substitution et/ou par addition et/ou suppression d'un ou plusieurs acides aminés d'un polypeptide de l'invention, et plus particulièrement de la séquence peptidique (II) décrite ci-dessus) ou d'une sous-séquence peptidique issue de cette dernière, entre le cadre de la présente invention, dès lors que cette modification n'altère pas les propriétés antigéniques dudit polypeptide.

L'invention concerne également les anticorps polyclonaux ou monoclonaux, susceptibles de reconnaître spécifiquement les polypeptides de l'invention, et de former des complexes immunologiques avec ces derniers.

Les anticorps polyclonaux de l'invention sont obtenus par immunisation d'un animal avec les polypeptides de l'invention, suivie de la récupération des anticorps formés.

Les anticorps monoclonaux de l'invention sont produits par tout hybridome susceptible d'être formé, par des méthodes classiques, à partir des cellules spléniques d'un animal, notamment de souris ou de rat, immunisés contre l'un des polypeptides purifiés de l'invention, d'une part et des cellules d'une lignée de cellules d'un myélome approprié d'autre part, et d'être sélectionné, par sa capacité à produire des anticorps monoclonaux reconnaissant le polypeptide initialement mis en oeuvre pour l'immunisation des animaux.

L'invention concerne également un procédé de détection (ou méthode de diagnostic) in vitro de la présence éventuelle de bactéries du genre Salmonella dans un échantillon biologique susceptible de les contenir, caractérisé en ce qu'il comprend :
- le cas échéant, la mise en culture de l'échantillon,
- le cas échéant, l'amplification du nombre de copies de la séquence nucléique à détecter à l'aide d'un couple d'amorces nucléiques telles que définies ci-dessus,
- la mise en contact de l'échantillon avec une sonde nucléique telle que définie ci-dessus, dans les conditions d'hybridation sus-mentionnées,
- la détection éventuelle de complexes d'hybridation formés entre la sonde sus-mentionnée et la séquence nucléique à détecter.

L'étape de mise en culture de l'échantillon biologique est avantageusement réalisée de la manière suivante : 25 g de l'échantillon biologique sont mis en culture dans 200 ml de bouillon nutritif dans un erlen-meyer de 1 litre durant 18 h à 37°C avec agitation.

L'étape d'amplification de la séquence nucléique à détecter, dans le procédé sus-mentionné, comprend avantageusement les étapes suivantes :
- une étape d'extraction de l'acide nucléique à détecter appartenant au génome des bactéries du genre Salmonella éventuellement présentes dans l'échantillon biologique, et,le cas échéant, une étape de traitement à l'aide d'une transcriptase inverse dudit acide nucléique si ce dernier est sous forme d'ARN,
- un cycle comprenant les étapes suivantes :
   . étape de dénaturation de l'acide nucléique double brin à détecter , ce qui conduit à la formation d'un acide nucléique simple brin ; cette étape est avantageusement réalisée à 94°C pendant 120 secondes,
   . étape de réassociation par hybridation de chacun des brins d'acide nucléique, obtenus lors de l'étape de dénaturation précédente, avec au moins une amorce telle que définie ci-dessus, par mise en contact des brins sus-mentionnés avec au moins un couple d'amorces sus-mentionnées ; cette étape est avantageusement réalisée à 68°C pendant 180 secondes,
   . étape d'élongation par formation à partir des amorces des ADN complémentaires aux brins sur lesquels elles sont hybridées en présence d'une ADN polymérase et de quantités appropriées des quatre nucléosides triphosphate différents (dATP, dCTP, dGTP, dTTP), ce qui conduit à la formation d'un plus grand nombre d'acides nucléiques double brin à détecter qu'à l'étape de dénaturation précédente ; cette étape est avantageusement réalisée à 72°C pendant 90 secondes, ce cycle étant répété un nombre de fois déterminé (de préférence entre 20 et 30 fois) pour obtenir ladite séquence nucléique à détecter éventuellement présente dans l'échantillon biologique dans une proportion suffisante pour permettre sa détection.

D'autres méthodes d'amplification et/ou de détection des séquences nucléiques caractéristiques des Salmonella, utilisant les sondes, et le cas échéant, les amorces nucléiques décrites ci-dessus, sont utilisables dans le cadre de la présente invention. A ce titre on peut citer la méthode décrite dans la demande de brevet internationale WO 85/06700, ou encore celle décrite dans la demande de brevet européen n° 0357336.

L'invention a également pour objet des kits pour la mise en oeuvre d'un procédé de détection in vitro de bactéries du genre Salmonella tel que décrit ci-dessus, ces kits comprenant :
- le cas échéant, un milieu approprié pour la mise en culture de l'échantillon biologique,
- au moins un couple d'amorces nucléiques décrites ci-dessus,
- le cas échéant, des réactifs appropriés à la mise en oeuvre du cycle d'amplification, notamment de l'ADN (ou ARN) polymérase, et des quantités appropriées des 4 nucléosides triphosphate différents,
- une (ou plusieurs) sonde(s) nucléique(s) telle(s) que décrite(s) ci-dessus, pouvant être marquée, capable de s'hybrider avec la (ou les) séquence(s) nucléique(s) à détecter,
- des réactifs appropriés à la mise en oeuvre de la réaction d'hybridation entre la (ou les) sonde(s) et la (ou les) séquence(s) nucléique(s) à détecter susmentionnées.
- un tissu ou fluide biologique de référence dépourvu de séquences nucléiques susceptibles de s'hybrider avec la (ou les) sonde(s) sus-mentionnée(s).

L'invention a également pour objet un procédé de détection in vitro de la présence éventuelle de bactéries de genre Salmonella dans un échantillon biologique susceptible de les contenir, caractérisé en ce qu'il comprend :
- le cas échéant, la mise en culture de l'échantillon biologique de la manière indiquée ci-dessus,
- le cas échéant, l'amplification du nombre de copies de la séquence nucléique correspondant selon le code génétique universel au polypeptide à détecter (cette amplification étant réalisée suivant le cycle d'amplification décrit ci-dessus),
- la mise en contact de l'échantillon sus-mentionné avec des anticorps de l'invention susceptibles de former un complexe immunologique avec le (ou les) polypeptide(s) à détecter,
- la détection éventuelle des complexes immunologiques formés entre lesdits anticorps et la (ou les) séquence(s) peptidique(s) à détecter.

L'invention concerne également des kits pour la mise en oeuvre du procédé de détection décrit ci-dessus, qui comprennent :
- le cas échéant, un milieu approprié pour la mise en culture de l'échantillon biologique,
- le cas échéant, un couple d'amorces décrites ci-dessus et des réactifs appropriés à la mise en oeuvre du cycle d'amplification, notamment de l'ADN (ou ARN) polymérase et des quantités appropriées des 4 nucléosides triphosphate différents,
- des anticorps, polyclonaux ou monoclonaux, pouvant être marqués, notamment de manière radioactive ou enzymatique, susceptibles de former un complexe immunologique avec la (ou les) séquence(s) peptidique(s) à détecter,
- les réactifs pour la constitution du milieu propice à la réalisation de la réaction immunologique entre les anticorps et la (ou les) séquence(s) peptidiques susmentionnées,
- les réactifs permettant la détection des complexes immunologiques formés lors de la susdite réaction immunologique. De tels réactifs peuvent également porter un marqueur ou être susceptibles d'être reconnus à leur tour par un réactif marqué,
- un tissu ou fluide biologique de référence dépourvu de polypeptides susceptibles d'être reconnus par les anticorps sus-mentionnés.

L'invention concerne également un procédé de préparation des séquences nucléiques décrites ci-dessus, ce procédé comprenant les étapes suivantes :
- incubation de l'ADN génomique, isolé à partir d'une bactérie Salmonella Typhi, par traitement par la soude à pH 12,5
- traitement de l'ADN ainsi extrait par une endonucléase appropriée,
- le clonage des acides nucléiques ainsi obtenus dans un vecteur approprié et la récupération de l'acide nucléique recherché à l'aide d'une sonde appropriée choisie parmi celles décrites ci-dessus.

Un procédé de préparation particulièrement avantageux des séquences nucléiques de l'invention comprend les étapes suivantes :
- la synthèse d'ADN en utilisant la méthode automatisée des β-cyanethyl phosphoramidite décrite dans Bioorganic Chemistry 4; 274-325 (1986),
- le clonage des acides nucléiques ainsi obtenus dans un vecteur approprié et la récupération de l'acide nucléique par hybridation avec une sonde appropriée choisie parmi celles décrites ci-dessus.

Un autre procédé de préparation des séquences nucléotidiques de l'invention comprend les étapes suivantes :
- l'assemblage d'oligonucléotides synthétisés chimiquement, pourvus à leurs extrémités de sites de restriction différents, dont les séquences sont compatibles avec l'enchaînement en acides aminés du polypeptide naturel selon le principe décrit dans Proc. Natl. Acad. Sci. USA, 80; 7461-7465, (1983),
- le clonage des acides nucléiques ainsi obtenus dans un vecteur approprié et la récupération de l'acide nucléique recherché par hybridation avec une sonde appropriée choisie parmi celles décrites ci-dessus.

L'invention a également pour objet tout acide nucléique recombinant contenant au moins une séquence nucléique de l'invention, insérée dans un acide nucléique hétérologue vis-à-vis de ladite séquence nucléique.

L'invention concerne plus particulièrement un acide nucléique recombinant tel que défini ci-dessus, dans lequel la séquence nucléique de l'invention est précédée d'un promoteur (notamment un promoteur inductible) sous le contrôle duquel la transcription de ladite séquence est susceptible d'être effectuée et, le cas échéant, suivie d'une séquence codant pour des signaux de terminaison de la transcription.

L'invention concerne tout vecteur recombinant, utilisé en particulier pour le clonage d'une séquence nucléique de l'invention, et/ou l'expression du polypeptide codé par cette séquence, et caractérisé en ce qu'il contient un acide nucléique recombinant, tel que défini ci-dessus, en l'un de ses sites non essentiel pour sa réplication.

A titre d'exemple de vecteur sus-mentionné, on citera les plasmides, les cosmides, ou les phages.

L'invention a également pour objet un procédé de préparation d'un polypeptide de l'invention, par transformation d'un hôte cellulaire à l'aide d'un vecteur recombinant de type sus-indiqué, suivie de la mise en culture de l'hôte cellulaire ainsi transformé, et de la récupération du polypeptide dans le milieu de culture.

Ainsi, l'invention concerne tout hôte cellulaire transformé par un vecteur recombinant tel que défini ci-dessus, et comprenant les éléments de régulation permettant l'expression de la séquence nucléique codant pour un polypeptide selon l'invention.

La présente invention concerne plus particulièrement un procédé de préparation d'un polypeptide de l'invention comprenant les étapes suivantes :
- le cas échéant, l'amplification de la quantité de séquences de nucléotides codant pour ledit polypeptide à l'aide de deux amorces d'ADN choisies de manière à ce que l'une de ces amorces soit identique aux 10 à 30 premiers nucléotides de la séquence nucléotidique codant pour ledit polypeptide, tandis que l'autre amorce est complémentaire des 10 à 30 derniers nucléotides (ou s'hybride avec ces 10 à 30 derniers nucléotides) de ladite séquence nucléotidique, ou inversement de manière à ce ce que l'une de ces amorces soit identique aux 10 à 30 derniers nucléotides de ladite séquence, tandis que l'autre amorce est complémentaire des 10 à 30 premiers nucléotides (ou s'hybride avec les 10 à 30 premiers nucléotides) de ladite séquence nucléotidique, suivie de l'introduction desdites séquences de nucléotides ainsi amplifiées dans un vecteur approprié,
- la mise en culture, dans un milieu de culture approprié, d'un hôte cellulaire préalablement transformé par un vecteur approprié contenant un acide nucléique selon l'invention comprenant la séquence nucléotidique codant pour ledit polypeptide, et
- la récupération, à partir du susdit milieu de culture du polypeptide produit par ledit hôte cellulaire transformé.

Les peptides selon l'invention peuvent être préparés par les techniques classiques, dans le domaine de la synthèse des peptides. Cette synthèse peut être réalisée en solution homogène ou en phase solide.

Par exemple, on aura recours à la technique de synthèse en solution homogène décrite par HOUBENWEYL dans l'ouvrage intitulé "Methode der Organischen Chemie" (Méthode de la Chimie Organique) édité par E. Wunsch, vol. 15-I et II., THIEME, Stuttgart 1974.

Cette méthode de synthèse consiste à condenser successivement deux-à-deux les aminoacyles successifs dans l'ordre requis, ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-aminopropyl)carbodiimide.

Lorsque l'aminoacyle mis en oeuvre possède une fonction acide supplémentaire (notamment dans le cas de l'acide glutamique), ces fonctions seront protégées, par exemple par des groupes t-butylester.

Dans le cas de la synthèse progressive, acide aminé par acide aminé, la synthèse débute de préférence par la condensation de l'amino-acide C-terminal avec l'aminoacide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Chem. Soc., 45, 2149-2154).

Pour fabriquer une chaîne peptidique selon le procédé de MERRIFIELD, on a recours à une résine polymère très poreuse, sur laquelle on fixe le premier acide aminé C-terminal de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième acide aminé qui fournit le second amino-acyle de la séquence recherchée, à partir du résidu amino-acyle C-terminal sur la fonction amine déprotégée du premier acide aminé C-terminal fixé sur la chaîne. De préférence, la fonction carboxyle de ce deuxième acide aminé est activée, par exemple par la dicyclohexylcarbodiimide, et la fonction amine est protégée, par exemple par le t-butyloxycarbonyle.

On obtient ainsi la première partie de la chaîne peptidique recherchée, qui comporte deux acide aminés, et dont la fonction amine terminale est protégée. Comme précédemment, on déprotège la fonction amine et on peut ensuite procéder à la fixation du troisième aminoacyle, dans les conditions analogues à celles de l'addition du deuxième acide aminé C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents acides aminés constituant la chaîne peptidique et on détache le peptide de la résine par exemple à l'aide d'acide fluorydrique.

L'invention sera davantage illustrée à l'aide des exemples contenus dans la description détaillée qui suit, cette dernière ne révêtant aucun caractère limitatif.

### 1. Isolement du fragment SacI de 2 Kb

Le but de la présente invention est le clonage du système adhésion-invasion de Typhi.

La souche utilisée est la souche Ty 2 de Typhi déposée à la Collection de L'INSTITUT PASTEUR sous la référence CIP 55-35.

Partant d'une collection de 2000 mutants indépendants obtenus par insertion d'un transposon dérivé de Tn5 (MANOIL C. et BECKWITH J., 1985. TnPhoA:a transposon probe for protein export signals. Proc. Natl. Acad. Sci. USA, 82, 8129-8133) dans le génome de la souche Typhi Ty 2, il a été obtenu un mutant qui n'était plus invasif dans le modèle de cellules HeLa en culture. La technique d'infection des cellules HeLa en culture par Typhi est la suivante:les cellules HeLa sont cultivées en milieu RPMI 1640 contenant 10 % de sérum de veau foetal. Ces cellules sont infectées par Typhi à un ratio de 100 bactéries par cellule. Après une heure d'incubation à 37°C, les cellules sont lavées avec le milieu de culture, puis remises en culture en présence de gentamycine à 100 µg/ml. Les bactéries intracellulaires sont détectées après 24 h par une coloration de Giemsa.

Après digestion du génome de ce mutant par l'endonucléase de restriction SacI, des expériences d'hybridation sur feuille de nitrate de cellulose ont montré que le transposon était inséré dans un fragment SacI de 2 kilobases (Kb).

Comme le transposon utilisé code pour la résistance à la kanamycine, le fragment SacI de 2 Kb, contenant le transposon a pu être cloné dans le site SacI du plasmide pUC18 (VIEIRA J. et MESSING J., 1982. The PUC plasmids, an M13mp7- derived system for insertion mutagenesis and sequencing with synthetic universal primers. Gene, 19, 259-268). C'est en utilisant ce plasmide recombinant que les séquences nucléiques faisant l'objet de la présente d'invention ont été isolées.

L'ADN génomique de la souche Ty 2 a été digéré par l'endonucléase SacI et les produits de cette restriction ont été ligaturés dans le site SacI du vecteur de clonage pUC18. Ce mélange de ligation a été utilisé pour transformer la souche Escherichia coli HB101 (déposée à la Collection de l'INSTITUT PASTEUR sous la référence CIP 102400). La sélection des transformants a été effectuée sur gélose nutritive contenant 100 µg/ml d'ampicilline. Après incubation durant 24 heures à l'étuve à 37°C, les transformants ont été repiqués sur des disques de nitrate de cellulose déposés sur des géloses nutritives contenant 100 µg/ml d'ampicilline. En vue de procéder à une hybridation in situ, ces disques ont ensuite été traités par une solution de dénaturation (NaOH 0,5M), puis par une solution de neutralisation (acétate d'ammonium IM, NaOH 0,02M). L'ADN libéré et dénaturé par ces traitements a été fixé sur les disques de nitrate de cellulose par chauffage à 80°C durant 3 heures.

Ces disques de nitrate de cellulose ainsi préparés ont été hybridés avec le fragment SacI de 2 kb contenant le transposon et marqué au ³²P. Les hybridations ont été réalisées à 65°C durant 24 heures en 6 x SSC (1 x SSC est constitué de 0,15 M NaCl et 0,015M citrate trisodique à pH 7) en milieu de Denhardt (0,1 % Ficoll ; 0,1 % polyvynil-pyrrolidone ; 0,1 % de sérum albumine de boeuf).

Il a été ainsi isolé un clone E. coli HB101 contenant un plasmide recombinant constitué du vecteur de clonage pUC18 dans lequel était inséré un fragment SacI de 2 kb (délimité par les sites S1 et S2 dans la carte de restriction de ce fragment qui est donnée dans la figure 1 en trait épais).

### 2. Région génétique associée au fragment SacI de 2 kb.

L'ADN génomique de la souche. Ty 2 a été digéré par l'endonucléase HindIII. Les produits de digestion, séparés par électrophorèse en gel d'agarose, ont été transférés par capillarité sur feuille de nitrate de cellulose. Ils ont ensuite été hybridés dans les conditions décrites ci-dessus avec le fragment SacI-BamH1 de 1,1 kb (fragment A sur la figure 1) ou avec le fragment HindIII-EcoR1 de 1,3 kb (fragment B sur la figure 1) qui sont tous les deux internes au fragment SacI de 2 kb.

Il a été constaté que le fragment SacI de 2 kb était recouvert par deux fragments HindIII de taille respectivement égale à 2,3 kb et 5,6 kb sur le génome de la souche Typhi Ty 2 (soit un fragment de 7,9 kb délimité par les sites H₁ et H₂ sur la carte de restriction enzymatique de cette région qui est donnée dans la figure 1).

### 3. Isolement de la sonde pour Salmonella.

L'ADN du plasmide recombinant contenant le fragment SacI de 2 kb a été restreint par les endonucléases SacI et HindIII. Cette double digestion libère un fragment SacI-HIndIII de 487 paires de bases, qui a été recloné entre les sites de restriction SacI et HindIII du vecteur de clonage pUC18.

Ce fragment SacI-HindIII de 487 paires de bases est appelé ci-après "Sonde pour Salmonella" et correspond à la séquence nucléique (I) définie ci-dessus. La position de ce fragment SacI-HindIII dans le fragment SacI de 2 kb est indiquée sur la figure 1.

### 4. Contrôle de la spécificité de la sonde pour Salmonella.

Pour effectuer ce contrôle, les souches utilisées ont été cultivées durant 24 heures à 37°C dans 200 µl de bouillon nutritif en plaques pour microtitration à 96 puits. A l'aide d'un inoculateur multi-points, ces souches sont remises en culture durant 5 heures à 37°C sur une feuille de nitrate de cellulose disposée sur une boite de gélose nutritive. La feuille de nitrate de cellulose est ensuite traitée et utilisée pour des expériences d'hybridation in situ.

Pour préparer la sonde pour Salmonella, le plasmide - recombinant (pUC18 contenant le fragment SacI-HindIII de 487 paires de bases) a été digéré par les endonucléases SacI et HindIII. Après séparation des produits de cette digestion par électrophorèse en gel d'agarose, la sonde pour Salmonella a été purifiée par électro-élution. Elle a, ensuite, été marquée au ³²P par déplacement de césure.

Les expériences d'hybridation ont été réalisées à 65°C en 6 x SSC et en milieu de Denhardt pendant 18 heures de réaction. Dans ces conditions expérimentales, la spécificité de la sonde pour Salmonella a été contrôlée avec 768 souches bactériennes, qui se répartissent en 384 souches n'appartenant pas au genre Salmonella et 384 appartenant au genre Salmonella et représentant les sept sous-espèces du genre Salmonella. Ces 768 souches proviennent de la Collection du Centre Collaborateur de l'OMS de Référence et de Recherche pour les Salmonella et de la collection du Centre National pour les Salmonella.

Aucune des 384 souches n'appartenant pas au genre Salmonella n'a répondu avec la sonde pour Salmonella.

Sur les 384 souches de Salmonella, 382 ont répondu avec la sonde pour Salmonella. Les deux souches n'ayant pas répondu avec la sonde pour Salmonella appartiennent, l'une au sérotype Wedding et l'autre au sérotype Zuerich. Il s'agit dans les deux cas de la souche de référence du sérotype. Pour ces deux souches, il a été vérifié qu'elles sont non-invasives dans le modèle sur cellules HeLa en culture et qu'elles ne possédent ni le fragment homologue de la sonde pour Salmonella , ni même le fragment SacI de 2 kb.

### 5. Détection de Salmonella dans les produits biologiques après amplification génique.

Dans un produit biologique, on ne peut aujourd'hui détecter une bactérie appartenant à une espèce donnée parmi plusieurs dizaines de millions d'autres bactéries. Pour pouvoir mettre en évidence la présence d'une Salmonella dans un produit biologique polymicrobien à l'aide d'une sonde nucléotidique, il est indispensable de procéder :
. à une amplification du nombre de bactéries à détecter en mettant en culture l'échantillon à analyser ;
. et à une amplification du nombre de copies de la cible que l'on veut détecter avec la sonde nucléotidique en utilisant la technique de l'amplification génique (ou technique de la "Polymerase Chain Reaction" ou PCR).

Pour les Salmonella, le fragment SacI-HindIII de 487 paires de bases (la sonde pour Salmonella) est spécifique du genre Salmonella. Si, à partir de l'échantillon biologique à analyser, on met en évidence la présence de ce fragment, on pourra en déduire que l'échantillon étudié est contaminé par Salmonella.

### 5.1. Description des deux amorces utilisées pour la PCR.

A partir de la séquence nucléotidique (I) de la sonde pour Salmonella, deux amorces utilisées pour mettre en oeuvre la technique d'amplification génique ont été sélectionnées.

Les enchaînements de 17 bases correspondant aux deux amorces, désignées SS-1 et SS-2 dans la description ci-dessus, ont été utilisés pour amplifier le nombre de copies pour Salmonella.

### 5.2. Protocole utilisé avec les amorces SS-1 et SS-2 pour la PCR.

Ce protocole é été mis au point en utilisant le kit "Gene amp" (marque déposée) de Perkin Elmer Cetus (réf. N 801-0055). Les amorces SS-1 et SS-2 ont été utilisées à la concentration finale de 200 pmole. Le mélange réactionnel a été réalisé selon les instructions du fabricant en utilisant 10 µl de la solution contenant l'ADN à amplifier sous un volume final de 50 µl. Ce mélange a été soumis à 20 cycles d'amplification en utilisant l'appareil "DNA thermal cycler" (marque déposée) de Perkin Elmer Cetus (réf. N801-0177) dans les conditions suivantes :
. étape de dénaturation à 94°C durant 120 secondes,
. étape de réassociation à 68°C durant 180 secondes,
. et étape d'élongation à 72°C durant 90 secondes.

L'ADN ainsi amplifié a été visualisé par électrophorèse (120 volts durant 30 minutes) en gel d'agarose contenant 2 % d'agarose à bas point de fusion (BRL ; réf. 5517) et % d'agarose normal (Sigma ; réf. A-6877). Après électrophorèse, l'ADN a été transféré sur feuille de nitrate de cellulose et hybridé avec la sonde pour Salmonella marquée au ³²P.

### 5.3. Spécificité et sensibilité de la technique

La souche LT2 de Salmonella sérotype Typhimurium, déposée à la Collection de l'Institut Pasteur sous la référence CIP 60.62T et la souche HB101 de E.coli (CIP 102.400) ont été utilisées pour étudier la spécificité et la sensibilité de la technique PCR dans les conditions expérimentales décrites ci-dessus.

La solution contenant l'ADN à amplifier a été préparée de la façon suivante :
1) les bactéries entières sont mises en suspension dans 100 µl d'eau distillée ;
2) la suspension bactérienne est traitée durant 10 minutes à 100°C dans un microtube pour centrifugation;
3) elle est ensuite centrifugée durant 3 minutes minutes à la vitesse maximale dans une microcentrifugeuse. En évitant le culot de centrifugation, on prélève 10 µl de cette solution pour procéder à l'amplification génique.

Lorsqu'on utilise une culture pure de la souche LT2 de Salmonella sérotype Typhimurium, on détecte l'ADN de 10 ou de moins de 10 bactéries dans 10 µl de solution soumise à l'amplification. Lorsqu'on utilise la souche HB101 de E. coli, aucune amplification n'est détectable même lorsqu'on travail avec l'ADN correspondant à 10⁶ bactéries dans 10 µl de solution soumise à l'amplification. Lorsqu'on réalise un mélange des deux souches bactériennes, on détecte l'ADN de 100 ou moins de 100 Salmonella mélangé avec l'ADN de 10⁶ E.coli HB101 dans 10 µl de la solution soumise à amplification.

### 5.4. Essai de détection de Salmonella dans un échantillon reconstitué au laboratoire.

L'échantillon utilisé est un lot de viande hachée destinée à l'alimentation animale. Il est très fortement contaminé par des bactéries à Gram positif ou négatif, aérobies ou anaérobies. Un comptage approximatif effectué sous microscope permet d'évaluer le nombre total de bactéries à 10⁹ par gramme de viande hachée. En numération sur boîtes de gélose nutritive, le nombre de bactéries aérobies cultivant sur ce milieu a été trouvé égal à 10⁸ par gramme de viande hachée. Cet échantillon ne contient pas de Salmonella : trois analyses effectuées avec les techniques classiques de bactériologie alimentaire se sont révélées négatives.

### 5.4.1. Préparation de l'échantillon pour analyse par PCR.

L'échantillon a été reconstitué en ajoutant un nombre variable de Salmonella sérotype Typhimurium (10, 10², 10³, 10⁴, 10⁵ ou 10⁶) à 25 g de viande hachée. Cet échantillon reconstitué est mis en culture dans 200 ml de bouillon trypto-caséine soja dans un erlen-meyer de 1 litre durant 18 heures à 37°C avec agitation. On traite de façon identique 25 g de viande hachée sans Salmonella.

Le lendemain, l'erlen-meyer est laissé durant 15 minutes sur la paillasse, sans agitation, à la température du laboratoire de façon à laisser sédimenter les plus gros débris. On prélève alors en surface 1 ml de bouillon que l'on transvase dans un microtube pour centrifugation et que l'on centrifuge durant 3 minutes à la vitesse maximale dans une microcentrifugeuse. Le surnageant est complètement éliminé à l'aide d'une pipette Pasteur. Le culot bactérien est remis parfaitement en suspension dans 100 µl d'eau distillée, puis passé durant 10 minutes à 100°C. On centrifuge de nouveau durant 3 minutes à la vitesse maximale. En évitant le culot de centrifugation, on prélève 10 µl de cette solution pour procéder à l'amplification génique dans les conditions déjà indiquées.

### 5.4.2. Spécificité et sensibilité de la technique.

Lorsqu'on part de 25 grammes d'échantillon sans Salmonella, on ne peut détecter aucune amplification de l'ADN contenu dans les 10 µl de la solution amplifiée. Lorsqu'on part de 25 grammes d'échantillon contenant 10² ou plus de 10² Salmonella, on détecte une amplification de l'ADN contenu dans les 10 µl de la solution amplifiée.

## Revendications

1. Séquence nucléique caractérisée en ce qu'elle contient tout ou partie de la séquence de 7,9 kb délimitée par les deux sites HindIII, désignés par H₁ et H₂ sur la carte de restriction de ladite séquence représentée sur la figure 1 de la souche Salmonella Typhi Ty2 et contenant l'information génétique nécessaire à l'activité d'infection in vitro des cellules HeLa en culture.

2. Séquence nucléique selon la revendication 1, caractérisée en ce qu'elle comprend la séquence de 2 kb délimitée par les deux sites SacI, désignés par S₁ et S₂ sur la carte de restriction représentée sur la figure 1 ou une partie de cette séquence nécessaire à l'activité d'infection in vitro de cellules HeLa en culture.

3. Séquence nucléique comportant toute ou partie de l'information génétique nécessaire à l'activité d'infection in vitro des cellules HeLa en culture par les bactéries du genre Salmonella, et susceptible de s'hybrider avec une séquence nucléique selon l'une des revendications 1 ou 2, dans des conditions stringentes.

4. Séquence nucléique constituée d'une succession d'environ 200 à 500 nucléotides, issue d'une séquence selon l'une des revendications 1 à 3, et utilisable en tant que sonde pour la détection in vitro de bactéries du genre Salmonella.

5. Sonde nucléique selon la revendication 4, caractérisée par l'enchaînement nucléotidique (I) suivant :

1
5'> CAATTACGCCCCGATTATTATATCTCCGGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGGACACAAAGCAAATTAGCGAGCTA
AATTCGATTGACAGTACCATGGTCTACTTACGCGGTAAGCATGAG
TTAAATCAATACACCCCCTATAGCTTACAGCAAGCGCTTAAATTG
CTGACTCAATGCGTTAATATGTCGCCAAACAGCATTGCGCCTTAC
TGTGCGCTGGCAGAATGCTACCTCAGCATGGCGCAAATGGGATT
TTTGATAAACAAAACGCAATGATCAAAGCTAAAGAACATGCGATT
AAGGCGACAGAGCTGGACCACAATAATCCACAAGCTT < 3'
587

6. Sonde nucléique selon la revendication 4 ou la revendication 5, caractérisée en ce qu'elle correspond selon le code génétique universel à la séquence en acides aminés (II) suivante :

GlnLeuArgProAspTyrTyrIleSerGlyGlnMetIleProAsp
GlyAsnAspAsnIleValGlnIleGluIleValArgValLysGly
TyrHisLeuLeuHisGlnGluSerIleLysLeuIleGluHisGln
ProAlaSerLeuLeuGlnAsnLysIleAlaAsnLeuLeuLeuArg
CysIleProGlyLeuArgTrpAspThrLysGlnIleSerGluLeu
AsnSerIleAspSerThrMetValTyrLeuArgGlyLysHisGlu
LeuAsnGlnTyrThrProTyrSerLeuGlnGlnAlaLeuLysLeu
LeuThrGlnCysValAsnMetSerProAsnSerIleAlaProTyr
CysAlaLeuAlaGluCysTyrLeuSerMetAlaGlnMetGlyIle
PheAspLysGlnAsnAlaMetIleLysAlaLysGluHisAlaIle
LysAlaThrGluLeuAspHisAsnAsnProGlnAla

7. Séquence nucléique constituée d'une succession d'environ 10 à 30 nucléotides issue d'une séquence selon l'une des revendications 1 à 6, et utilisable en tant qu'amorce nucléique pour l'amplification génique d'une séquence selon l'une des revendications 1 à 5.

8. Séquences nucléiques selon la revendication 7, utilisables pour l'amplification de la séquence nucléique délimitée par les nucléotides situés aux positions 26 et 469 de l'enchaînement nucléotidique (I) selon la revendication 6 ou la revendication 7, caractérisée par les enchaînements nucléotidiques suivants :

SS-1
5'>GGT CCA GCT CTG TCG CC<3'

SS-2
5'>CCG GGC AGA TGA TAC CC<3'

9. Polypeptide correspondant selon le code génétique universel à une séquence nucléique selon l'une quelconque des revendications 1 à 8.

10. Polypeptide constitué par toute ou partie de la séquence en acides aminés (II) suivante, cette séquence correspondant selon le code génétique universel à la séquence nucléique (I) définie dans la revendication 5 :

GlnLeuArgProAspTyrTyrIleSerGlyGlnMetIleProAsp
GlyAsnAspAsnIleValGlnIleGluIleValArgValLysGly
TyrHisLeuLeuHisGlnGluSerIleLysLeuIleGluHisGln
ProAlaSerLeuLeuGlnAsnLysIleAlaAsnLeuLeuLeuArg
CysIleProGlyLeuArgTrpAspThrLysGlnIleSerGluLeu
AsnSerIleAspSerThrMetValTyrLeuArgGlyLysHisGlu
LeuAsnGlnTyrThrProTyrSerLeuGlnGlnAlaLeuLysLeu
LeuThrGlnCysValAsnMetSerProAsnSerIleAlaProTyr
CysAlaLeuAlaGluCysTyrLeuSerMetAlaGlnMetGlyIle
PheAspLysGlnAsnAlaMetIleLysAlaLysGluHisAlaIle
LysAlaThrGluLeuAspHisAsnAsnProGlnAla

11. Anticorps, polyclonaux ou monoclonaux, susceptibles de reconnaître spécifiquement un polypeptide selon la revendication 9 ou la revendication 10.

12. Procédé de détection in vitro de la présence éventuelle de bactéries du genre Salmonella dans un échantillon biologique susceptible de les contenir caractérisé en ce qu'il comprend :
- le cas échéant, la mise en culture de l'échantillon,
- le cas échéant, l'amplification du nombre de copies de la séquence nucléique à détecter à l'aide d'un couple d'amorces nucléiques selon la revendication 7 ou la revendication 8,
- la mise en contact de l'échantillon avec une sonde nucléique selon l'une des revendications 4 à 6, dans les conditions d'hybridation définies dans la revendication 4,
- la détection éventuelle de complexes d'hybridation formés entre la sonde sus-mentionnée et la séquence nucléique à détecter.

13. Procédé selon la revendication 12, caractérisé en ce que l'étape d'amplification de la séquence nucléique à détecter, comprend les étapes suivantes :
- une étape d'extraction de l'acide nucléique à détecter appartenant au génome des bactéries du genre Salmonella éventuellement présentes dans l'échantillon biologique, et, le cas échéant, une étape de traitement à l'aide d'une transcriptase inverse dudit acide nucléique si ce dernier est sous forme d'ARN,
- un cycle comprenant les étapes suivantes :
. dénaturation de l'acide nucléique double brin à détecter, ce qui conduit à la formation d'un acide nucléique simple brin,
. hybridation de chacun des brins d'acide nucléique, obtenus lors de l'étape de dénaturation précédente, avec au moins une amorce selon la revendication 7 ou la revendication 8, par mise en contact des brins sus-mentionnés avec au moins un couple d'amorces susmentionnées,
. formation à partir des amorces des ADN complémentaires aux brins sur lesquels elles sont hybridées en présence d'une ADN polymérase et de quantités appropriées des quatre nucléosides triphosphate (dNTP) différents, ce qui conduit à la formation d'un plus grand nombre d'acides nucléiques double brin à détectter qu'à l'étape de dénaturation précédente, ce cycle étant répété un nombre de fois déterminé pour obtenir ladite séquence nucléique à détecter éventuellement présente dans l'échantillon biologique dans une proportion suffisante pour permettre sa détection.

14. Kit pour la mise en oeuvre d'un procédé selon la revendication 12 ou la revendication 13, caractérisé en ce qu'il comprend :
- le cas échéant, un milieu approprié pour la mise en culture de l'échantillon biologique,
- au moins un couple d'amorces nucléiques selon la revendication 7 ou la revendication 8,
- le cas échéant, des réactifs appropriés à la mise en oeuvre du cycle d'amplification, notamment de l'ADN polymérase, et des quantités appropriées des 4 nucléosides triphosphate différents,
- une (ou plusieurs) sonde(s) nucléique(s) selon l'une des revendications 4 à 6, pouvant être marquée, capable de s'hybrider avec la (ou les) séquence(s) nucléique(s) à détecter,
- des réactifs appropriés à la mise en oeuvre de la réaction d'hybridation entre la (ou les) sonde(s) et la (ou les) séquence(s) nucléique(s) à détecter susmentionnées.
- un tissu ou fluide biologique de référence dépourvu de séquences nucléiques susceptibles de s'hybrider avec la (ou les) sonde(s) sus-mentionnée(s).

15. Procédé de détection in vitro de la présence éventuelle de bactéries de genre Salmonella dans un échantillon biologique susceptible de les contenir, caractérisé en ce qu'il comprend :
- le cas échéant, la mise en culture de l'échantillon biologique,
- le cas échéant, l'amplification, selon le procédé défini dans la revendication 13, du nombre de copies de la (ou les) séquence(s) nucléique(s) correspondant selon le code génétique universel au(x) polypeptide(s) à détecter,
- la mise en contact de l'échantillon sus-mentionné avec des anticorps selon la revendication 11,
- la détection éventuelle des complexes immunologiques formés entre lesdits anticorps et la (ou les) séquence(s) peptidique(s) à détecter.

16. Kit pour la mise en oeuvre d'un procédé selon la revendication 15, caractérisé en ce qu'il comprend :
- le cas échéant, un milieu approprié pour la mise en culture de l'échantillon biologique,
- le cas échéant, un couple d'amorces nucléiques selon la revendication 7 ou la revendication 8, et des réactifs appropriés à la mise en oeuvre du cycle d'amplification, notamment de l'ADN polymérase et des quantités appropriées des 4 nucléosides triphosphate différents,
- des anticorps, polyclonaux ou monoclonaux, selon la revendication 11, pouvant être marqués, notamment de manière radioactive ou enzymatique, susceptibles de former un complexe immunologique avec la (ou les) séquence(s) peptidique(s) à détecter,
- les réactifs pour la constitution du milieu propice à la réalisation de la réaction immunologique entre les anticorps et la (ou les) séquence(s) peptidique(s) sus-mentionnés,
- les réactifs permettant la détection des complexes immunologiques formés lors de la susdite réaction immunologique,
- un tissu ou fluide biologique de référence dépourvu de polypeptides susceptibles d'être reconnus par les anticorps sus-mentionnés.

17. Acide nucléique recombinant contenant au moins une séquence nucléique selon l'une des revendications 1 à 8, insérée dans un acide nucléique hétérologue vis-à-vis de ladite séquence nucléique.

18. Acide nucléique recombinant selon la revendication 17 caractérisé en ce que la séquence nucléique selon l'une des revendications 1 à 8 est précédée d'un promoteur (notamment un promoteur inductible) sous le contrôle duquel la transcription de ladite séquence est susceptible d'être effectuée et, le cas échéant, suivie d'une séquence codant pour des signaux de terminaison de la transcription.

19. Vecteurs recombinants, notamment des plasmides, cosmides, ou phages, contenant un acide nucléique recombinant selon la revendication 17 ou la revendication 18, en l'un de leurs sites non essentiels pour leur réplication.

20. Procédé de préparation d'un polypeptide selon la revendication 9 ou la revendication 10, par transformation d'un hôte cellulaire à l'aide d'un vecteur recombinant selon la revendication 19, suivie de la mise en culture de l'hôte cellulaire ainsi transformé, et de la récupération du polypeptide dans le milieu de culture.

21. Procédé de préparation d'un polypeptide selon la revendication 9 ou la revendication 10, comprenant les étapes suivantes :
- le cas échéant, l'amplification de la quantité de séquences de nucléotides codant pour ledit polypeptide à l'aide de deux amorces d'ADN selon la revendication 7 ou la revendication 8, choisies de manière à ce que l'une de ces amorces soit identique aux 10 à 30 premiers nucléotides de la séquence nucléotidique codant pour ledit polypeptide, tandis que l'autre amorce est complémentaire des 10 à 30 derniers nucléotides (ou s'hybride avec ces 10 à 30 derniers nucléotides) de ladite séquence nucléotidique, ou inversement de manière à ce que l'une de ces amorces soit identique aux 10 à 30 derniers nucléotides de ladite séquence, tandis que l'autre amorce est complémentaire des 10 à 30 premiers nucléotides) de ladite séquence nucléotidique, suivie de l'introduction desdites séquences de nucléotides ainsi amplifiées dans un vecteur approprié,
- la mise en culture, dans un milieu de culture approprié, d'un hôte cellulaire préalablement transformé par un vecteur selon la revendication 19,
- la récupération, à partir du susdit milieu de culture du polypeptide produit par ledit hôte cellulaire transformé.

## Patentansprüche

1. Nukleinsäuresequenz,
dadurch gekennzeichnet, daß sie die Gesamtheit oder einen Teil der Sequenz von 7,9 kb umfaßt, die von den zwei HindIII-Stellen, die durch H₁ und H₂ in der in Figur 1 dargestellten Restriktionskarte der Sequenz auf dem Stamm Salmonella Typhi Ty2 bezeichnet werden, begrenzt wird und die die genetische Information enthält, die für die Aktivität einer in vitro-Infektion der HeLa-Zellen in Kultur notwendig ist.

2. Nukleinsäuresequenz nach Anspruch 1,
dadurch gekennzeichnet, daß sie die Sequenz von 2 kb, die von den zwei SacI-Stellen, die durch S₁ und S₂ in der in Figur 1 dargestellten Restriktionskarte bezeichnet werden, begrenzt wird, oder einen Teil dieser Sequenz umfaßt, die für die Aktivität einer in vitro-Infektion von HeLa-Zellen in Kultur notwendig ist.

3. Nukleinsäuresequenz, die die Gesamtheit oder einen Teil der genetischen Information trägt, die für die Aktivität einer in vitro-Infektion der HeLa-Zellen in Kultur durch die Bakterien der Gattung Salmonella notwendig ist, und die in der Lage ist, mit einer Nukleinsäuresequenz nach einem der Ansprüche 1 oder 2 unter stringenten Bedingungen zu hybridisieren.

4. Nukleinsäuresequenz, die durch eine Aufeinanderfolge von ungefähr 200 bis 500 Nukleotiden gebildet wird, die von einer Sequenz nach einem der Ansprüche 1 bis 3 abstammt und als Sonde für den in vitro-Nachweis von Bakterien der Gattung Salmonella verwendet werden kann.

5. Nukleinsäuresonde nach Anspruch 4,
gekennzeichnet durch die folgende Aneinanderreihung von Nukleotiden (I):

1
5'> CAATTACGCCCCGATTATTATATCTCCGGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGGACACAAAGCAAATTAGCGAGCTA
AATTCGATTGACAGTACCATGGTCTACTTACGCGGTAAGCATGAG
TTAAATCAATACACCCCCTATAGCTTACAGCAAGCGCTTAAATTG
CTGACTCAATGCGTTAATATGTCGCCAAACAGCATTGCGCCTTAC
TGTGCGCTGGCAGAATGCTACCTCAGCATGGCGCAAATGGGGATT
TTTGATAAACAAAACGCAATGATCAAAGCTAAAGAACATGCGATT
AAGGCGACAGAGCTGGACCACAATAATCCACAAGCTT < 3'
587

6. Nukleinsäuresonde nach Anspruch 4 oder Anspruch 5, gekennzeichnet dadurch, daß sie gemäß dem universellen genetischen code der folgenden Aminosäuresequenz (II) entspricht:

GlnLeuArgProAspTyrTyrIleSerGlyGlnMetIleProAsp
GlyAsnAspAsnIleValGlnIleGluIleValArgValLysGly
TyrHisLeuLeuHisGlnGluSerIleLysLeuIleGluHisGln
ProAlaSerLeuLeuGlnAsnLysIleAlaAsnLeuLeuLeuArg
CysIleProGlyLeuArgTrpAspThrLysGlnIleSerGluLeu
AsnSerIleAspSerThrMetValTyrLeuArgGlyLysHisGlu
LeuAsnGlnTyrThrProTyrSerLeuGlnGlnAlaLeuLysLeu
LeuThrGlnCysValAsnMetSerProAsnSerIleAlaProTyr
CysAlaLeuAlaGluCysTyrLeuSerMetAlaGlnMetGlyIle
PheAspLysGlnAsnAlaMetIleLysAlaLysGluHisAlaIle
LysAlaThrGluLeuAspHisAsnAsnProGlnAla

7. Nukleinsäuresequenz,
die durch eine Aufeinanderfolge von ungefähr 10 bis 30 Nukleotiden gebildet wird, die von einer Sequenz nach einem der Ansprüche 1 bis 6 abstammt und die als Nukleinsäurestartsequenz für die Genamplifikation einer Sequenz nach einem der Ansprüche 1 bis 5 verwendet werden kann.

8. Nukleinsäuresequenzen nach Anspruch 7,
die für die Amplifikation der Nukleinsäuresequenz verwendet werden können, die durch die an den Positionen 26 und 469 der Nukleotidaneinanderreihung (I) nach Anspruch 6 oder Anspruch 7 befindlichen Nukleotide begrenzt wird, und die durch die folgenden Nukleotidaneinanderreihungen gekennzeichnet sind:

SS-1
5'>GGT CCA GCT CTG TCG CC<3'

SS-2
5'>CCG GGC AGA TGA TAC CC<3'

9. Polypeptid,
das gemäß dem universellen genetischen Code einer Nukleinsäuresequenz nach einem der Ansprüche 1 bis 8 entspricht.

10. Polypeptid,
das durch die Gesamtheit oder einen Teil der folgenden Aminosäuresequenz (II) gebildet wird, wobei diese Sequenz gemäß dem universellen genetischen Code der in Anspruch 5 definierten Nukleinsäuresequenz (I) entspricht:

GlnLeuArgProAspTyrTyrIleSerGlyGlnMetIleProAsp
GlyAsnAspAsnIleValGlnIleGluIleValArgValLysGly
TyrHisLeuLeuHisGlnGluSerIleLysLeuIleGluHisGln
ProAlaSerLeuLeuGlnAsnLysIleAlaAsnLeuLeuLeuArg
CysIleProGlyLeuArgTrpAspThrLysGlnIleSerGluLeu
AsnSerIleAspSerThrMetValTyrLeuArgGlyLysHisGlu
LeuAsnGlnTyrThrProTyrSerLeuGlnGlnAlaLeuLysLeu
LeuThrGlnCysValAsnMetSerProAsnSerIleAlaProTyr
CysAlaLeuAlaGluCysTyrLeuSerMetAlaGlnMetGlyIle
PheAspLysGlnAsnAlaMetIleLysAlaLysGluHisAlaIle
LysAlaThrGluLeuAspHisAsnAsnProGlnAla

11. Polyklonale oder monoklonale Antikörper, die spezifisch ein Polypeptid nach Anspruch 9 oder Anspruch 10 erkennen können.

12. Verfahren zum in vitro-Nachweis der etwaigen Anwesenheit von Bakterien der Gattung Salmonella in einer biologischen Probe, die diese möglicherweise enthält,
dadurch gekennzeichnet, daß es umfaßt:
- gegebenenfalls die Probe in Kultur zu nehmen,
- gegebenenfalls die Anzahl von Kopien der nachzuweisenden Nukleinsäuresequenz mittels eines Paares Nukleinsäurestartsequenzen nach Anspruch 7 oder Anspruch 8 zu amplifizieren,
- die Probe mit einer Nukleinsäuresonde nach einem der Ansprüche 4 bis 6 unter den in Anspruch 4 definierten Hybridisierungsbedingungen in Kontakt zu bringen,
- zwischen der vorstehend genannten Sonde und der nachzuweisenden Nukleinsäuresequenz gebildete Hybridisierungskomplexe gegebenenfalls nachzuweisen.

13. Verfahren nach Anspruch 12,
dadurch gekennzeichnet, daß der Schritt der Amplifizierung der nachzuweisenden Nukleinsäuresequenz die folgenden Schritte umfaßt:
- einen Schritt einer Extraktion der nachzuweisenden Nukleinsäure, die aus dem Genom der Bakterien der Gattung Salmonella stammt, die möglicherweise in der biologischen Probe anwesend sind, und gegebenenfalls einen Schritt einer Behandlung dieser Nukleinsäure mittels einer inversen Transkriptase, sofern die Nukleinsäure in Form von RNA vorliegt,
- einen Zyklus, der die folgenden Schritte umfaßt:
. eine Denaturierung der nachzuweisenden doppelsträngigen Nukleinsäure, was zur Bildung einer einzelsträngigen Nukleinsäure führt,
. eine Hybridisierung jedes der aufgrund des vorangegangenen Denaturierungsschritts erhaltenen Nukleinsäurestränge mit mindestens einer Startsequenz nach Anspruch 7 oder Anspruch 8, indem die vorstehend genannten Stränge mit mindestens einem Paar vorstehend genannter Startsequenzen in Kontakt gebracht werden,
. ausgehend von den Startsequenzen eine Bildung zu den Strängen komplementärer DNAs, mit denen sie hybridisiert sind, in Gegenwart einer DNA-Polymerase und geeigneter Mengen der vier verschiedenen Nukleosidtriphosphate (dNTP), was zu der Bildung einer größeren Anzahl von nachzuweisenden doppelsträngigen Nukleinsäuren führt als bei dem vorangegangenen Denaturierungsschritt, wobei dieser Zyklus eine festgelegte Anzahl von Malen wiederholt wird, um die nachzuweisende Nukleinsäuresequenz, die möglicherweise in der biologischen Probe anwesend ist, in einer ausreichenden Menge, um deren Nachweis zu ermöglichen, zu erhalten.

14. Kit zum Ausführen eines Verfahrens nach Anspruch 12 oder Anspruch 13,
dadurch gekennzeichnet, daß er enthält:
- gegebenenfalls ein geeignetes Medium, um die biologische Probe in Kultur zu nehmen,
- mindestens ein Paar Nukleinsäurestartsequenzen nach Anspruch 7 oder Anspruch 8,
- gegebenenfalls geeignete Reagenzien, um den Amplifizierungszyklus durchzuführen, insbesondere DNA-Polymerase und geeignete Mengen der 4 verschiedenen Nukleosidtriphosphate,
- eine (oder mehrere) Nukleinsäuresonde(n) nach einem der Ansprüche 4 bis 6, die markiert sein können, die mit der (oder den) nachzuweisenden Nukleinsäuresequenz(en) hybridisieren können,
- geeignete Reagenzien, um die Hybridisierungsreaktion zwischen der (oder den) Sonde(n) und der (oder den) vorstehend genannten nachzuweisenden Nukleinsäuresequenz(en) zu bewerkstelligen,
- ein biologisches Referenzgewebe oder eine biologische Referenzflüssigkeit, das bzw. die frei ist von Nukleinsäuresequenzen, die mit der (oder den) vorstehend genannten Sonde(n) hybridisieren können.

15. Verfahren zum in vitro-Nachweis der etwaigen Anwesenheit von Bakterien der Gattung Salmonella in einer biologischen Probe, die diese möglicherweise enthält,
dadurch gekennzeichnet, daß es umfaßt:
- gegebenenfalls die biologische Probe in Kultur zu nehmen,
- gegebenenfalls nach dem in Anspruch 13 definierten Verfahren die Anzahl von Kopien der Nukleinsäuresequenz(en), die gemäß dem universellen genetischen Code dem oder den nachzuweisenden Polypeptid(en) entspricht bzw. entsprechen, zu amplifizieren,
- die vorstehend genannte Probe mit Antikörpern nach Anspruch 11 in Kontakt zu bringen,
- die zwischen den Antikörpern und der (oder den) nachzuweisenden Peptidsequenz(en) gebildeten immunologischen Komplexe gegebenenfalls nachzuweisen.

16. Kit zum Ausführen eines Verfahrens nach Anspruch 15, dadurch gekennzeichnet, daß er enthält:
- gegebenenfalls ein geeignetes Medium, um die biologische Probe in Kultur zu nehmen,
- gegebenenfalls ein Paar Nukleinsäurestartseqenzen nach Anspruch 7 oder Anspruch 8 und geeignete Reagenzien, um den Amplifizierungszyklus durchzuführen, insbesondere DNA-Polymerase und geeignete Mengen der 4 verschiedenen Nukleosidtriphosphate,
- polyklonale oder monoklonale Antikörper nach Anspruch 11, die markiert sein können, insbesondere radioaktiv oder enzymatisch, geignet für eine immunologische Reaktion zu bilden, mit der (oder den) nachzuweisenden Peptidsequenz (en),
- die Reagenzien für die Konstitution des zur Ausführung der immunologischen Reaktion zwischen den Antikörpern und der (oder den) vorstehend genannten Peptidsequenz(en) geeigneten Mediums,
- die Reagenzien, die den Nachweis der aufgrund der vorstehend genannten immunologischen Reaktion gebildeten immunologischen Komplexe erlauben,
- ein biologisches Referenzgewebe oder eine biologische Referenzflüssigkeit, das bzw. die frei ist von Polypeptiden, die von den vorstehend genannten Antikörpern erkannt werden können.

17. Rekombinante Nukleinsäure, die mindestens eine Nukleinsäuresequenz nach einem der Ansprüche 1 bis 8 enthält, die in eine hinsichtlich der genannten Nukleinsäuresequenz heterologe Nukleinsäure insertiert ist.

18. Rekombinante Nukleinsäure nach Anspruch 17,
dadurch gekennzeichnet, daß der Nukleinsäuresequenz nach einem der Ansprüche 1 bis 8 ein Promotor (insbesondere ein induzierbarer Promotor) vorangeht, unter dessen Kontrolle die Transkription der genannten Sequenz bewirkt werden kann, und dieser Nukleinsäuresequenz gegebenenfalls eine Sequenz folgt, die Transkriptionsterminationssignale kodiert.

19. Rekombinante Vektoren, insbesondere Plasmide, Cosmide oder Phagen, die eine rekombinante Nukleinsäure nach Anspruch 17 oder Anspruch 18 in einer ihrer Stellen, die für ihre Replikation nicht essentiell sind, enthalten.

20. Verfahren zur Herstellung eines Polypeptids nach Anspruch 9 oder Anspruch 10 durch Transformation eines zellulären Wirts mittels eines rekombinanten Vektors nach Anspruch 19, gefolgt von dem In-Kultur-Nehmen des so transformierten zellulären Wirts und von der Gewinnung des Polypeptids in dem Kulturmedium.

21. Verfahren zur Herstellung eines Polypeptids nach Anspruch 9 oder Anspruch 10, das die folgenden Schritte umfaßt:
- gegebenenfalls die Amplifizierung der Menge von Nukleotidsequenzen, die das Polypeptid kodieren, mittels zweier DNA-Startsequenzen nach Anspruch 7 oder Anspruch 8, die so ausgewählt werden, daß eine dieser Startsequenzen identisch mit 10 bis 30 ersten Nukleotiden der Nukleotidsequenz ist, die das genannte Polypeptid kodiert, wohingegen die andere Startsequenz zu den 10 bis 30 letzten Nukleotiden der Nukleotidsequenz komplementär ist (oder mit diesen 10 bis 30 letzten Nukleotiden der Nukleotidsequenz hybridisiert) oder umgekehrt, daß eine dieser Startsequenzen mit 10 bis 30 letzten Nukleotiden dieser Sequenz identisch ist, wohingegen die andere Startsequenz zu den 10 bis 30 ersten Nukleotiden der Nukleotidsequenz komplementär ist, gefolgt von der Einführung der genannten so amplifizierten Nukleotidsequenzen in einen geeigneten Vektor,
- das In-Kultur-Nehmen eines vorab mit einem Vektor nach Anspruch 19 transformierten zellulären Wirts in einem geeigneten Kulturmedium,
- die Gewinnung des von dem transformierten zellulären Wirt produzierten Polypeptids aus dem vorstehend genannten Kulturmedium.

## Claims

1. Nucleic sequence, characterized in that it contains all or part of the 7.9 kb sequence delimited by the two HindIII sites, designated H₁ and H₂ on the restriction map of the said sequence represented in Figure 1, from the strain Salmonella Typhi Ty2, and containing the genetic information necessary for the activity of infecting in vitro HeLa cells in culture.

2. Nucleic sequence according to Claim 1, characterized in that it comprises the 2 kb sequence delimited by the two SacI sites, designated S₁ and S₂ on the restriction map represented in Figure 1, or part of this sequence which is necessary for the activity of infecting in vitro HeLa cells in culture.

3. Nucleic sequence comprising all or part of the genetic information necessary for the activity of infecting in vitro HeLa cells in culture with bacteria of the genus Salmonella, and capable of hybridizing with a nucleic sequence according to one of Claims 1 or 2, under stringent conditions.

4. Nucleic sequence consisting of a succession of about 200 to 500 nucleotides, which sequence is derived from a sequence according to one of Claims 1 to 3 and can be used as probe for the in vitro detection of bacteria of the genus Salmonella.

5. Nucleic probe according to Claim 4, characterized by the following nucleotide chain (I):

1
5'> CAATTACGCCCCGATTATTATATCTCCGGGCAGATGATACCCGAT
GGTAATGATAATATTGTACAGATCGAGATAGTTCGGGTTAAAGGT
TATCACCTGCTGCACCAGGAAAGCATTAAGTTGATAGAACACCAA
CCCGCTTCTCTCTTGCAAAACAAAATTGCGAATCTTTTGCTCAGA
TGTATTCCCGGACTTCGCTGGGACACAAAGCAAATTAGCGAGCTA
AATTCGATTGACAGTACCATGGTCTACTTACGCGGTAAGCATGAG
TTAAATCAATACACCCCCTATAGCTTACAGCAAGCGCTTAAATTG
CTGACTCAATGCGTTAATATGTCGCCAAACAGCATTGCGCCTTAC
TGTGCGCTGGCAGAATGCTACCTCAGCATGGCGCAAATGGGGATT
TTTGATAAACAAAACGCAATGATCAAAGCTAAAGAACATGCGATT
AAGGCGACAGAGCTGGACCACAATAATCCACAAGCTT < 3'
587

6. Nucleic probe according to Claim 4 or Claim 5, characterized in that it corresponds, according to the universal genetic code, to the following amino acid sequence (II):

GlnLeuArgProAspTyrTyrIleSerGlyGlnMetIleProAsp
GlyAsnAspAsnIleValGlnIleGluIleValArgValLysGly
TyrHisLeuLeuHisGlnGluSerIleLysLeuIleGluHisGln
ProAlaSerLeuLeuGlnAsnLysIleAlaAsnLeuLeuLeuArg
CysIleProGlyLeuArgTrpAspThrLysGlnIleSerGluLeu
AsnSerIleAspSerThrMetValTyrLeuArgGlyLysHisGlu
LeuAsnGlnTyrThrProTyrSerLeuGlnGlnAlaLeuLysLeu
LeuThrGlnCysValAsnMetSerProAsnSerIleAlaProTyr
CysAlaLeuAlaGluCysTyrLeuSerMetAlaGlnMetGlyIle
PheAspLysGlnAsnAlaMetIleLysAlaLysGluHisAlaIle
LysAlaThrGluLeuAspHisAsnAsnProGlnAla

7. Nucleic sequence consisting of a succession of about 10 to 30 nucleotides derived from a sequence according to one of Claims 1 to 6, and which can be used as nucleic primer for the gene amplification of a sequence according to one of Claims 1 to 5.

8. Nucleic sequences according to Claim 7, which can be used for the amplification of the nucleic sequence delimited by the nucleotides situated at positions 26 and 469 of the nucleotide chain (I) according to Claim 6 or Claim 7, characterized by the following nucleotide chains:

SS-1
5'>GGT CCA GCT CTG TCG CC<3'

SS-2
5'>CCG GGC AGA TGA TAC CC<3'

9. Polypeptide corresponding, according to the universal genetic code, to a nucleic sequence according to any one of Claims 1 to 8.

10. Polypeptide consisting of all or part of the following amino acid sequence (II), this sequence corresponding, according to the universal genetic code, to the nucleic sequence (I) defined in Claim 5:

GlnLeuArgProAspTyrTyrIleSerGlyGlnMetIleProAsp
GlyAsnAspAsnIleValGlnIleGluIleValArgValLysGly
TyrHisLeuLeuHisGlnGluSerIleLysLeuIleGluHisGln
ProAlaSerLeuLeuGlnAsnLysIleAlaAsnLeuLeuLeuArg
CysIleProGlyLeuArgTrpAspThrLysGlnIleSerGluLeu
AsnSerIleAspSerThrMetValTyrLeuArgGlyLysHisGlu
LeuAsnGlnTyrThrProTyrSerLeuGlnGlnAlaLeuLysLeu
LeuThrGlnCysValAsnMetSerProAsnSerIleAlaProTyr
CysAlaLeuAlaGluCysTyrLeuSerMetAlaGlnMetGlyIle
PheAspLysGlnAsnAlaMetIleLysAlaLysGluHisAlaIle
LysAlaThrGluLeuAspHisAsnAsnProGlnAla

11. Polyclonal or monoclonal antibodies which are capable of recognizing specifically a polypeptide according to Claim 9 or Claim 10.

12. Process for the in vitro detection of the possible presence of bacteria of the genus Salmonella in a biological sample capable of containing them, characterized in that it comprises:
- whore appropriate, the culturing of the sample,
- where appropriate, the amplification of the number of copies of the nucleic sequence to be detected with the aid of a pair of nucleic primers according to Claim 7 or Claim 8,
- the bringing of the sample into contact with a nucleic probe according to one of Claims 4 to 6, under hybridization conditions defined in claim 4,
- the possible detection of hybridization complexes formed between the abovementioned probe and the nucleic sequence to be detected.

13. Process according to Claim 12, characterized in that the step of amplifying the nucleic sequence to be detected comprises the following steps:
- a step of extracting the nucleic acid to be detected belonging to the genome of the bacteria of the Salmonella genus which may be present in the biological sample, and, where appropriate, a step of treating, with the aid of a reverse transcriptase, the said nucleic acid if the latter is in RNA form,
- a cycle comprising the following steps:
. denaturation of the double-stranded nucleic acid to be detected, which leads to the formation of a single-stranded nucleic acid,
. hybridization of each of the nucleic acid strands, obtained during the preceding denaturation step, with at least one primer according to Claim 7 or Claim 8, by bringing the abovementioned strands into contact with at least one pair of primers mentioned above,
. formation from the primers of the DNAs complementary to the strands to which they are hybridized in the presence of a DNA polymerase and appropriate quantities of four different nucleoside triphosphates (dNTPs), which leads to the formation of a larger number of double-stranded nucleic acids to be detected than at the preceding denaturation step, this cycle being repeated a determined number of times in order to obtain the said nucleic sequence to be detected, which may be present in the biological sample in a proportion sufficient to allow its detection.

14. Kit for carrying out. a process according to Claim 12 or Claim 13, characterized in that it comprises:
- where appropriate, a medium appropriate for culturing the biological sample,
- at least one pair of nucleic primers according to Claim 7 or Claim 8,
- where appropriate, reagents appropriate for carrying out the amplification cycle, especially DNA polymerase, and appropriate quantities of the four different nucleoside triphosphates,
- one (or more) nucleic probe(s) according to one of Claims 4 to 6, which may be labelled, capable of hybridizing with the nucleic sequence(s) to be detected,
- reagents appropriate for carrying out the hybridization reaction between the probe(s) and the nucleic sequence(s) to be detected which are mentioned above,
- a reference biological tissue or fluid free of nucleic sequences capable of hybridizing with the abovementioned probe(s).

15. Process for the in vitro detection of the possible presence of bacteria of the genus Salmonella in a biological sample capable of containing them, characterized in that it comprises:
- where appropriate, the culturing of the biological sample,
- where appropriate, the amplification, according to the process defined in claim 13, of the number of copies of the nucleic sequence(s) corresponding, according to the universal genetic code, to the polypeptide(s) to be detected,
- the bringing of the abovementioned sample into contact with antibodies according to Claim 11,
- the possible detection of the immunological complexes formed between the said antibodies and the peptide sequence (s) to be detected.

16. Kit for carrying out a process according to Claim 15, characterized in that it comprises:
- where appropriate, a medium appropriate for culturing the biological sample,
- where appropriate, a pair of nucleic primers according to Claim 7 or Claim 8, and reagents appropriate for carrying out the amplification cycle, especially DNA polymerase and appropriate quantities of the four different nucleoside triphosphates,
- polyclonal or monoclonal antibodies according to Claim 11, which may be labelled, specially radioactively or enzymatically, capable of forming an immunological complex with the peptide sequence(s) to be detected,
- the reagents for the preparation of the medium suitable for carrying out the immunological reaction between the antibodies and the peptide sequence(s) which are mentioned above,
- the reagents which allow the detection of the immunological complexes formed during the abovementioned immmological reaction,
- a reference biological tissue or fluid free of polypeptides capable of being recognized by the abovementioned antibodies.

17. Recombinant nucleic acid containing at least one nucleic sequence according to one of Claims 1 to 8, inserted into a nucleic acid which is heterologous in relation to the said nucleic sequence.

18. Recombinant nucleic acid according to Claim 17, characterized in that the nucleic sequence according to one of Claims 1 to 8 is preceded by a promoter (especially an inducible promoter) under whose control the transcription of the said sequence is capable of being carried out and, where appropriate, followed by a sequence encoding signals for termination of transcription.

19. Recombinant vectors, especially plasmids, cosmids or phages, containing a recombinant nucleic acid according to Claim 17 or Claim 18, in one of their sites which are non-essential for their replication.

20. Process for the preparation of a polypeptide according to Claim 9 or Claim 10, by transforming a cell host with the aid of a recombinant vector according to Claim 19, followed by the culture of the host cell thus transformed and the recovery of the polypeptide from the culture medium.

21. Process for the preparation of a polypeptide according to Claim 9 or Claim 10, comprising the following steps:
- where appropriate, the amplification of the quantity of nucleotide sequences encoding the said polypeptide with the aid of two DNA primers according to Claim 7 or Claim 8, which are chosen so that one of these primers is identical to the first 10 to 30 nucleotides of the nucleotide sequence encoding the said polypeptide, while the other primer is complementary to the last 10 to 30 nucleotides (or hybridizes with these last 10 to 30 nucleotides) of the said nucleotide sequence, or conversely so that one of these primers is identical to the last 10 to 30 nucleotides of the said sequence, while the other primer is complementary to the first 10 to 30 nucleotides of the said nucleotide sequence,
followed by the introduction of the said nucleotide sequences thus amplified into an appropriate vector,
- the culturing, in an appropriate culture medium, of a cell host previously transformed by a vector according to Claim 19,
- the recovery, from the abovementioned culture medium, of the polypeptide produced by the said transformed cell host.
